# EUROPÄISCHE PATENTANMELDUNG

(11) **EP 2 719 344 A1**
(43) Veröffentlichungstag der Anmeldung: **16.04.2014**
(21) Anmeldenummer: 12188164.3
(22) Anmeldetag: 11.10.2012
(51) Int. Cl.: A61B 17/3203, A61B 17/00, A61B 17/22, A61M 11/00, B05B 1/12

(54) **Fluidchirurgisches Instrument mit variablem Strahlbild**

(71) Anmelder: ERBE Elektromedizin GmbH, 72072 Tübingen (DE)
(72) Erfinder: Kühner, Ralf, 70567 Stuttgart (DE); Mitzlaff, Lothar, 8600-531 Lagos (PT)
(74) Vertreter: Rüger, Barthelt & Abel

(57) **Zusammenfassung**

Erfindungsgemäß wird ein fluidchirurgisches Instrument bereitgestellt, das eine schwingungsbeaufschlagbare Düse (20) aufweist. Diese Grundkonzept kann dazu genutzt werden, während des Betriebs der Düse die Strahlrichtung zu ändern, um eine künstliche Wischbewegung zu erzeugen oder um der Düse lediglich Mikrobewegungen zu erteilen, die in den ausgestoßenen Fluidstrahl einen Querimpuls einleiten und so zu einer Veränderung des Strahlbilds führen. Es ist möglich beide Effekte miteinander zu kombinieren, Auf diese Weise kann der Chirurg bspw. softwaretechnisch bereitgestellte verschiedene Strahlformen auf Befehl abrufen und mit ein und demselben Instrument erzeugen. Er kann somit verschiedene Wirkungen erzielen, ohne das Instrument und die Düse wechseln zu müssen.

## Beschreibung

Die Erfindung betrifft ein fluidchirurgisches Instrument zur chirurgischen Behandlung von Gewebe.

In der Wasserstrahlchirurgie wird ein Wasserstrahl z.B. zur Durchtrennung oder zum Wegspülen bzw. Auflösung von Gewebe eingesetzt. Dazu werden mittels eines wasserstrahlchirurgischen Instruments am distalen Ende desselben austretende Wasserstrahlen auf das biologische Gewebe appliziert. Abhängig von der kinetischen Energie und der erzeugten Strahlform können unterschiedliche Gewebeeffekte erzielt werden.

Gerade bei endoskopischen Instrumenten sind die Düse und deren Austrittsöffnung sehr klein. Schon geringste Geometrieabweichungen einer Düse können die gewünschte Strahlform jedoch nachhaltig beeinflussen. Die Form des Wasserstrahls ist dabei direkt an die Geometrie der Düse gebunden. Die Bereitstellung verschiedener Düsenformen zur Erzeugung unterschiedlicher Strahlformen und Behandlungseffekte ist somit ein logistisches Problem. Insbesondere bei Einweggeräten, die für lediglich einmaligen Gebrauch vorgesehen sind, ist häufig der hohe Aufwand für die Fertigung spezieller Düsen nicht gerechtfertigt. Außerdem ist ein Düsen- oder Instrumentenwechsel erforderlich, wenn der Chirurg eine andere Strahlform wünscht.

Beispiele für die Wasserstrahlchirurgie sind dem japanischen Gebrauchsmuster JP GM 60-192808, der DE 34 21 390, der DE 30 19 115 sowie der DE 37 15 418 A1 zu entnehmen. Exemplarisch sind dort in den Figuren 30, 31, 32 verschiedene Düsenformen veranschaulicht, die wahlweise zum Einsatz kommen können.

Insbesondere bei der Behandlung von Parenchymgewebe, beispielsweise beim Auflösen desselben, wird häufig gewünscht, zu erhaltende Strukturen, wie bspw. Blutgefäße, Nerven, Bindegewebe oder dergleichen, zu schonen. Hierzu ist es bspw. bekannt, mit einem fächerförmigen Strahl zu arbeiten, dessen Fluidtröpfchen für sich genommen relativ geringe Energie aufweisen, so dass weiches Gewebe aufgelöst, festeres Gewebe aber nicht angegriffen wird. Andererseits werden zuweilen gewebetrennende bzw. schneidende Funktionen benötigt, bei denen ein scharf gebündelter Strahl auf das Gewebe einwirkt.

Zur Erzeugung dieser unterschiedlichen Strahlformen werden üblicherweise unterschiedliche Düsen eingesetzt. Dazu muss der Chirurg das Instrument wechseln.

Es ist Aufgabe der Erfindung ein Konzept anzugeben, mit dem sich unterschiedliche Effekte bei der fluidchirurgischen Behandlung von Gewebe mit geringem Aufwand und möglichst unter Verkürzung der Operationszeit darstellen lassen.

Diese Aufgabe wird mit einem fluidchirurgischen Instrument nach Anspruch 1 gelöst:

Das erfindungsgemäße fluidchirurgische Instrument kann ein offenchirurgisch oder ein endoskopisch einzusetzendes Instrument sein. Jedenfalls weist es mindestens einen Fluidleiter auf, der einer Düse unter Druck stehendes Fluid, vorzugsweise eine NaCl-Lösung, zuleitet. Der Fluidleiter erstreckt sich dabei durch einen Träger, der bspw. durch einen Handgriff eines offenchirurgisch einzusetzenden Instruments oder durch ein längliches Element, z.B. einen Schlauch oder ein Rohr gebildet ist. Der Schlauch oder das Rohr können als Sonde durch ein Endoskop in einen Körper einführbar sein oder selbst ein Endoskop bilden.

Die Düse ist bezüglich des Trägers beweglich angeordnet, um eine Oszillationsbewegung auszuführen. Ein oder mehrere Aktuatoren dienen dabei dazu, die Düse gezielt oszillierend zu bewegen. Die Düse kann dabei quer zur Strahlrichtung in einer oder in zwei Richtungen oszillieren. Zusätzlich oder alternativ kann der Düse eine Schwenkbewegung um eine oder zwei quer zu der Strahlrichtung stehende Achsen erteilt werden. Es ist auch möglich, der Düse eine Taumelbewegung zu erteilen.

Der Aktuator, der die Düsenbewegung ermöglicht, kann Teil des Instruments selbst sein. Er ist dann nicht trennbar mit dem Instrument verbunden. Es ist auch möglich, dass der Aktuator eine vom Instrument getrennte Einheit bildet, die vom Instrument abnehmbar ausgebildet ist. Diese Aktuatoreinheit kann dann die Anforderungen an eine wiederaufbereitbare Einheit erfüllen.

Durch jede der genannten Maßnahmen wird das Strahlbild des aus der Düse austretenden linearen Strahls verändert. Es kann eine lineare streifenförmige Fläche, eine Kreisfläche, eine Mehreckfläche eine gerundete oder Ecken aufweisende gefüllte oder ringförmige Fläche sein.

Abhängig von der Amplitude und der Frequenz der Düsenbewegung können zumindest zwei Betriebsarten erreicht werden:

In einem ersten Modus wird die Düse in einem Winkelbereich geschwenkt, der so groß ist, wie der vom Strahl zu überstreichende Bereich. Dies entspricht einem künstlichen Tremor bzw. einer Wischbewegung, die der Chirurg sonst vornimmt, z.B. um großflächige Gewebeabtragungen vorzunehmen.

In einem zweiten Modus wird die Düse in einem Winkelbereich geschwenkt oder linear seitlich bewegt, der deutlich kleiner ist, als der von dem Strahl zu überstreichende Bereich. Die Bewegung der Düse ist dabei so schnell, dass dem Strahl ein zusätzlich zu dem in Strahlrichtung gerichteten Impuls ein oszillierender Querimpuls überlagert wird. Frequenz und Größe des Querimpulses bestimmen zusammen mit dem in Austrittsrichtung gerichteten Längsimpuls die Strahlform. Auf diese Weise können mit ein und derselben Düse verschiedene Strahlformen erzielt werden, bspw. ein gerader schneidender Strahl, wenn die Düse nicht oszilliert, ein Fächerstrahl, wenn die Düse in einer Querrichtung linear oder schwenkend oszilliert, eine Kegelhohlstrahl, wenn der Düse eine oszillierende Kreisbewegung erteilt wird, sowie ein Kegelvollstrahl, wenn die Düse durch entsprechende Ansteuersignale von in Querrichtung wirkenden Aktuatoren mit in Querrichtung gerichteten umlaufenden Impulsen wechselnder Größe beaufschlagt wird.

Im ersten Modus schwingt die Düse mit niedriger Frequenz und hoher Amplitude. Im zweiten Modus schwingt die Düse mit hoher Frequenz und niedriger Amplitude. Es sind Mischformen des ersten und zweiten Modus bei mittlerer Frequenz und mittlerer Amplitude möglich. Es ist auch möglich, den ersten und zweiten Modus zu überlagern. Ein Beispiel dafür ist, wenn mit der Düse ein schwenkender Fächerstrahl erzeugt wird. Es können alle anderen oben genannten Formen des ersten und zweiten Modus überlagert werden.

Der Träger, durch den sich der Fluidleiter erstreckt, kann, wie erwähnt, ein Handgriff oder ein starres Rohr oder auch ein Schlauch mit einer gewissen Flexibilität sein, der bspw. durch ein Endoskop in eine Körperhöhle eingeführt wird. Am distalen Ende dieses Trägers ist die Düse angeordnet. Zwischen dem Träger und der Düse ist mindestens ein Aktuator wirksam, so dass die Düse in Bezug auf den Träger kontrolliert bewegbar ist. Dazu kann die Düse an dem Träger z.B. elastisch gelagert sein. Zur Lagerung kann der Aktuator selbst dienen.

Als Aktuator zur oszillierenden Bewegung der Düse können Piezoaktuatoren, Vibramotoren oder Ähnliches vorgesehen werden. Damit lassen sich der vom Chirurgen verursachten Bewegung des Wasserstrahls Mikrobewegungen bzw. Mikroschwingungen überlagern. Somit kann mit ein und derselben Düsengeometrie, die bspw. durch eine zylindrische Bohrung gegeben sein kann, unterschiedliche Formen des applizierten Wasserstahls nahezu frei nach Wunsch gebildet bzw. "geformt" werden.

Durch die Erfindung wird es möglich, mit ein und derselben Düse verschiedenste Stahlformen zu erzeugen. Es wird die sonst vorhandene feste Bindung der Stahlform an die Düsengeometrie beseitigt. Auch komplizierte Strahlformen lassen sich mit einfachen Düsen erzeugen, die sonst lediglich dazu geeignet sind, einen schneidenden, d.h. dünnen laminaren Strahl mit hoher kinetischer Energie zu erzeugen. Damit werden der zur Bereitstellung von chirurgischen Instrumenten erforderliche Aufwand ebenso gesenkt wie die erforderliche Operationszeit, wenn während einer Operation unterschiedliche Strahlformen erforderlich sind. Insbesondere eignet sich die Erfindung zum Einsatz bei Einmalinstrumenten, deren Strahlform neben dem anliegenden Fluiddruck lediglich noch durch die an dem Aktuator anliegenden Ansteuersignale festgelegt werden.

Die Möglichkeit der Nutzung einfachster Düsengeometrien ermöglicht sehr kleine Bauformen der Düsen auch aus schwer zu bearbeitenden Materialien, wie bspw. hochtemperaturfesten Werkstoffen, z.B. Wolfram. Damit kann die Düse nicht nur als fluidchirurgisches Bauteil, sondern zugleich als Elektrode für elektrochirurgische Anwendungen eingesetzt werden. Auch dies trägt zur Verkürzung von Operationszeiten und zur Verbesserung der Qualität der chirurgischen Behandlung bei.

Dem Instrument ist vorzugsweise eine Ansteuereinrichtung für den Aktuator zugeordnet, um dem Aktuator entsprechend Ansteuersignale zuzuleiten, die mittels einer Auswahleinrichtung vorzugsweise umschaltbar sind, um verschiedene Behandlungseffekte zu erzielen, indem verschiedene Strahlbilder erzeugt werden. Die Ansteuereinrichtung kann Teil eines speisenden medizinischen Geräts sein. Alternativ kann die Ansteuereinrichtung in das Instrument eingebaut sein.

Im einfachsten Fall können der Düse durch den Aktuator kreisförmige Bewegungen erteilt werden, die einen zylindrischen oder einen kegelförmigen Strahl mit unterschiedlichen Durchmessern erzeugen. Eine rein translatorische Schwingung, vorzugsweise senkrecht zur Strömungsrichtung (oder auch in einem anderen Winkel orientiert), kann einen flächigen, fächerförmigen Strahl erzeugen.

Es ist möglich, lediglich die distal angeordnete Düse oder einen distalen Abschnitt des Instruments an einer entsprechenden Sonde oder eines Applikators in die gewünschte Strahlform erzeugende Schwingung zu versetzen oder auch einen längeren Schaftteil oder auch den Handgriff eines Instruments selbst in Schwingung zu versetzen, wobei der Schaft die Schwingung auf den distalen Abschnitt überträgt. Es können beide Maßnahmen kombiniert werden.

Zur Einstellung einer gewünschten Strahlform kann eine Auswahleinrichtung vorgesehen sein, die ein oder mehrere Bedienungselemente aufweist. Ein oder mehrere solcher Bedienungselemente können unmittelbar am Handgriff des chirurgischen Instruments oder einem speisenden medizinischen Gerät, das das unter Druck stehende Fluid bereitstellt oder anderweitig, bspw. an einem Fußschalter, angeordnet sein.

Vorzugsweise sind den verschiedenen Strahlformen oder Betriebsarten entsprechende Ansteuersignale zugeordnet, die durch in einem Speicher abgelegte Daten definiert werden und bedarfsweise abgerufen werden können. Solche vordefinierten Programme oder Daten erleichtern die Bedienung. Es ist aber auch möglich bspw. Frequenzamplitude und Kurvenform der Ansteuersignale in Grenzen frei wählbar bereitzustellen, so dass der Operateur die Strahlform wunschentsprechend einstellen kann, ohne an vordefinierte Strahlformen gebunden zu sein. Beispielsweise kann mit einer hohen Oszillationsfrequenz der Düse ein harter Strahl in einen Fächerstrahl verwandelt werden, der im Nahfeld der Düse die gleiche Wirkung, d.h. den gleichen Gewebeeffekt hat wie ein laminarer, d.h. harter Strahl und somit schneidend wirkt. Wegen des mit zunehmender Entfernung von der Düse eintretenden Verlusts an kinetischer Energie wird er jedoch mit zunehmendem Düsenabstand zunehmend weicher, wodurch er vom Gewebe und tiefer liegenden Gewebeschichten leicht absorbiert werden kann und somit keine Perforation mehr verursacht oder zumindest eine höhere Sicherheit gegen Perforation bietet.

Die Oszillation der Düse kann auch dazu genutzt werden, dem Strahl eine Wischbewegung zu überlagern. Beispielsweise kann dies das Wegspülen von Parenchymstrukturen mittels des Wasserstrahls erleichtern, um Blutgefäße, Nerven und Tumore freizulegen. Auf diese Weise verursacht der Wasserstrahl keine zu große Tiefenwirkung und damit keine Schädigung tiefer liegender, nicht sichtbarere Gewebestrukturen. Die sonst manuell auszuführende Wischbewegung kann durch das Oszillieren der Düse wie oben beschrieben ausgeführt werden. Gewissermaßen wird so ein künstlicher Tremor erzeugt. Idealerweise ist der eigentliche Handgriff vom Anwendungsteil mechanisch so entkoppelt, dass keine störenden Vibrationen auf den Anwender, d.h. den Operateur, übertragen werden. Wiederum können Intensität, Form und Richtung dieser Querbewegungen der Düse an dem speisenden Gerät und/oder an dem Instrument oder an einem sonstigen Bedienteil, wie bspw. Pedal, eingestellt werden.

Es ist möglich, die vorzugsweise mit geringerer Frequenz dafür größere Amplitude einer Wischbewegung der Düse mit der vorzugsweise höherfrequenten Schwingungsbewegung der Düse zur Erzeugung bestimmter Strahlformen zu kombinieren. Dazu könne ein oder mehrere Aktuatoren eingesetzt werden.

Das fluidchirurgische Instrument eignet sich, wie erwähnt, zur schonenden Parenchymauflösung, zur Freilegung von Tumoren, zur Behandlung von Wundheilungsstörungen, bei der großflächige Areale behandelt werden müssen. Die Kombination aus Steuerung der Strahlform und Steuerung der Wischbewegung ermöglicht ein gleichmäßigeres Behandeln großflächiger Gewebeareale und erleichtert im Vergleich zu einem laminaren Strahl, der manuell zu führen ist, die Durchführung der Operation erheblich.

Außerdem ist es möglich, den Wasserstrahl zu pulsen. Dies kann durch Einleitung von Druckschwingungen in den Fluidkanal bzw. Fluidleiter geschehen. Die Druckschwingungen können am proximalen Ende des Fluidkanals erzeugt werden. Vorzugsweise können sie aber mittels eines Piezoelements bspw. am distalen Ende in der Nähe der Düse generiert werden, so dass einzelne Tropfen des Trennmediums mit sehr hoher kinetischer Energie auf das Gewebe treffen. Damit lässt sich mit geringen Flüssigkeitsmengen eine relativ große Abtragungsleistung bzw. ein starker Effekt am Gewebe bewirken. Außerdem können z.B. an der Düse ein oder mehrere als Ventil wirkende Aktoren vorgesehen sein, um den Fluidstrahl aktiv zu unterbrechen und dadurch den Gewebeeffekt zu beeinflussen.

Der Träger kann, insbesondere bei endoskopischen Anwendungen, weitere Elemente, wie Lichtleiter, eine Optik, HF-chirurgische Mittel, z.B. mindestens eine Elektrode und mindestens eine elektrische Leitung, einen Absaugkanal und dergleichen enthalten.

Weiter Einzelheiten vorteilhafter Ausführungsformen der Erfindung ergeben sich aus der Zeichnung oder der Beschreibung. Es zeigen:
Figur 1 ein fluidchirurgisches Instrument bei der Behandlung eines Organs und ein speisendes Gerät für die offenchirurgische Behandlung, in jeweils äußerst schematisierter Darstellung,
Figur 2 und 3 Ausführungsbeispiele für das fluidchirurgische Instrument nach Figur 1, in schematisierter Prinzipdarstellung,
Figur 4 ein fluidchirurgisches Instrument zu endoskopischen Chirurgie, in schematisierter Darstellung,
Figur 5 ein Ausführungsbeispiel für ein fluidchirurgisches Instrument zur laproskopischen Anwendung, in schematisierter, ausschnittsweiser Längsschnittdarstellung,
Figur 6 eine abgewandelte Ausführungsform des Instruments nach Figur 5, in schematisierter, ausschnittsweiser Darstellung,
Figur 7 das Instrument nach Figur 6, in einer schematisierten Stirnansicht,
Figur 8 Ansteuersignale für die Aktuatoren des Instruments nach Figur 7 zur Erzeugung eines Vollkegelstrahls und
Figur 9 Größe und Orientierung des Querimpulses des Strahls bei der Erzeugung eines Vollkegels als Diagramm.

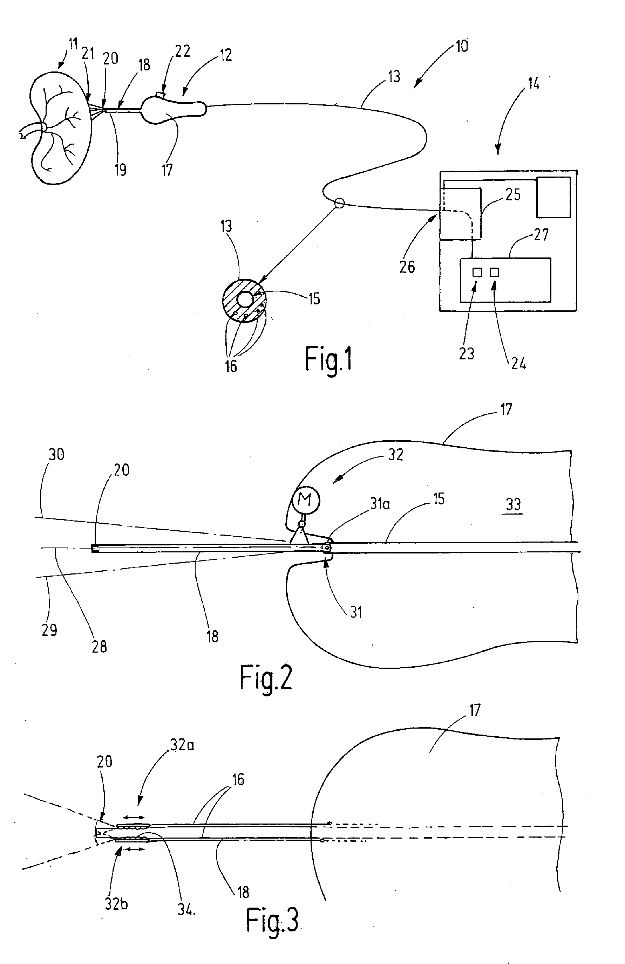

Das in Figur 1 veranschaulichte System 10 zur fluidchirurgischen Behandlung eines Organs 11 oder sonstigen biologischen Gewebes, umfasst ein fluidchirurgisches Instrument 12, das über eine Leitung 13 mit einem speisenden chirurgischen Gerät 14 verbunden ist. Die Leitung 13 enthält, die in Figur 1 zur Veranschaulichung im Querschnitt dargestellt ist, mindestens einen Fluidleiter oder Fluidkanal 15, der dem Instrument 12 unter Druck stehendes Fluid, bspw. Wasser (NaCl-Lösung) zuführt. Außerdem enthält die Leitung vorzugsweise mindestens eine oder mehrere elektrische Leitungen 16, die voneinander isoliert sind und dazu dienen, elektrische Leistung von dem Gerät 14 zu dem Instrument 12 zu übertragen. Dieses weist einen sich von seinem Handgriff 17 weg erstreckenden Applikator 18 auf, an dessen distalen Ende 19 eine Düse 20 angeordnet ist. Diese lässt einen Fluidstrahl 21 austreten, der in Figur 1 beispielhaft als Fächerstrahl oder Kegelstrahl dargestellt ist.

Das Instrument 12 enthält mindestens ein Bedienelement 22, mit dem beispielsweise die Form des Strahls 21 beeinflussbar ist. Das Bedienelement 22 kann, wie dargestellt, an dem Handgriff 17 angeordnet sein. Alternativ kann die Aktivierung des Instruments 12 sowie die Form und Stärke des Strahls 21 auch durch ein Bedienelement erfolgen, das in Form eines Fußschalters ausgebildet ist. Ergänzend oder alternativ können ein oder mehrere Bedienelemente 23, 24 an dem Gerät 14 angeordnet sein, die dazu geeignet sind, Eigenschaften des Strahls 21, insbesondere dessen Form, zu steuern. Dazu enthält das Gerät 14 eine Einrichtung 25 beispielsweise in Form einer Pumpe, mittels derer die Düse 20 über den Applikator 18 und die Leitung 13, dort insbesondere über den Fluidkanal 15, mit unter Druck stehendem Fluid versorgbar ist. Die Leitung 13 ist dazu mit ihrem proximalen Ende 26 an die Einrichtung 25 angeschlossen. Die elektrischen Leitungen 16 sind mit einer Ansteuereinrichtung 27 verbunden, mittels derer Ansteuerimpulse erzeugbar sind, die über die Leitungen 16 zu einem oder mehreren Aktoren gelangen, mittels derer die Düse 20 quer beweglich ist. Alternativ kann die Ansteuereinrichtung auch in dem Instrument 12 angeordnet und über die Leitungen 16 mit Strom versorgt werden. Ein HF-Generator 38 kann dazu vorgesehen sein, über eine der Leitungen 16 die Düse und/oder den Applikator 18 mit HF-Leistung zur Durchführung HF-chirurgischer Eingriffe zu beaufschlagen.

Figur 2 zeigt schematisch einen Aufbau mit beweglicher Düse 20. Die Düse 20 ist dort aus ihrer Mittelposition heraus in verschiedene Schwenkpositionen bewegbar. Dabei wird der gesamte Applikator 18 geschwenkt, bspw. um eine künstliche Wischbewegung zu erzielen. Die verschiedenen den jeweiligen Schwenkpositionen des Applikators 18 zugeordneten Längsrichtungen sind durch strichpunktierte Linien 28, 29, 30 symbolisiert. Der Schwenkwinkel kann relativ groß bemessen sein und z.B. dem zu überstreichenden Fächer entsprechen.

Der Applikator 18 kann mit dem übrigen Fluidkanal 15 über eine schwenkbare Verbindung 31 verbunden sein. Diese Verbindung 31 kann bspw. eine elastische Verbindung oder dergleichen sein.

Der rohrartige Applikator 18 ist mit einem Aktuator 32 verbunden, der dazu eingerichtet ist, den Applikator 18 und somit letztendlich die Düse 20 schwingend quer zur Längsrichtung 28 bzw. 29, 30 zu bewegen. Der Aktuator 32 ist dabei von dem Handgriff 17 getragen, der hier einen Träger 33 bildet, durch den sich der Fluidkanal 15 erstreckt. Der Abtrieb des Aktuators 32 wirkt seitlich auf den Applikator 18 ein. Der Aktuator 32 kann ein Piezoantrieb, ein Magnetantrieb, ein Vibramotor, ein Motor mit Exzenterantrieb für den Applikator 18 oder dergleichen sein.

Während der Aktuator 32 bei der Ausführungsform nach Figur 2 den gesamten Applikator 18 schwenkt, kann es auch zweckmäßig sein, lediglich einen kurzen Teil desselben oder die Düse 20 allein zu schwenken oder quer zu bewegen. Ein Ausführungsbeispiel veranschaulicht sehr schematisch Figur 3. Der Applikator 18 trägt hier an seinem Ende die Düse 20, deren elastische schwenkbare Ankopplung an den Applikator 18 durch einen Balg 34 symbolisiert ist. Anstelle eines Balgs können auch andere technische flexible Elemente bspw. ein Schlauch oder dergleichen, zur Anwendung kommen.

Um der Düse 20 ein Quervibration zu erteilen, kann der Balg 34 oder das sonstige bewegliche Element durch ein oder mehrer Aktuatoren 32a, 32b, hier bspw. längskontrahierende Piezoelemente, überbrückt sein, die jeweils mit einem Ende mit der Düse 20 und mit ihrem anderen Ende mit dem Applikator 18 verbunden sind. Wiederum dienen Leitungen 16 dazu, Ansteuersignale zu den Aktuatoren 32a, 32b zu leiten. Der Schwenkwinkel oder seitliche Bewegungsweg der Düse 20 kann deutlich geringer sein, als der von Strahl zu definierende Öffnungswinkel.

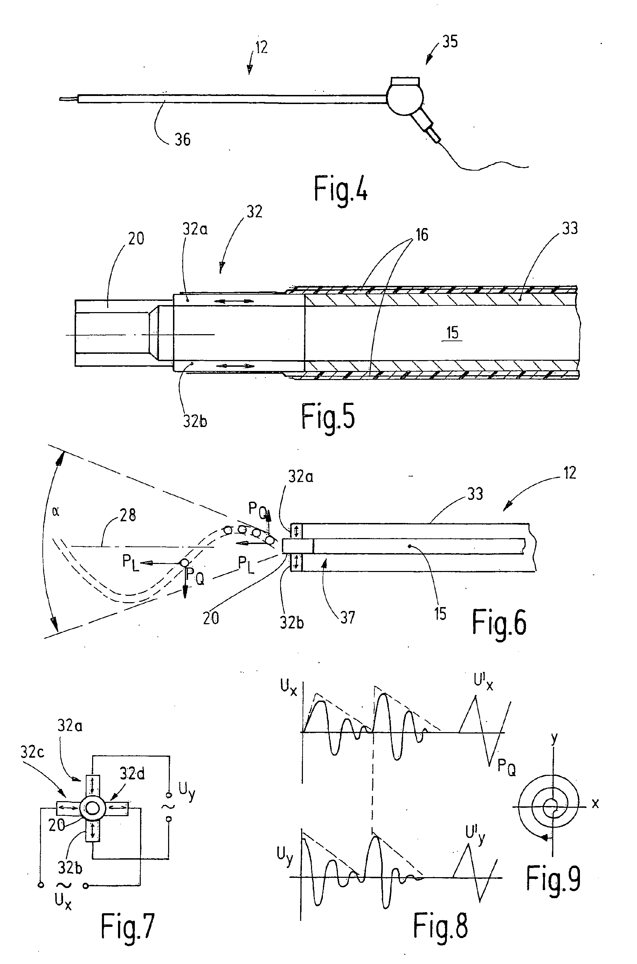

In ähnlicher Weise kann das Instrument 12 ausgebildet sein, wenn es für den Einsatz in einem Endoskop 35 eingerichtet sein soll. Dieses weist wie Figur 4 schematisch zeigt einen Rohrschaft 36 auf, durch den sich das Instrument 12 erstreckt. Figur 5 veranschaulicht einen entsprechenden flexiblen Träger 33, der hier als flexibles Rohr oder Schlauch ausgebildet ist und sowohl den Fluidkanal 15 wie auch die Leitungen 16 enthält. Endständig ist wiederum die Düse 20 vorgesehen. Die Düse 20 ist vorzugsweise mit einer Zylinderbohrung versehen, um einen laminaren harten Strahl zu erzeugen. Die Verbindung zwischen dem Träger 33 und der Düse 20 wird hier wieder durch eine Aktuatoranordnung 32 erbracht, zu der ein oder mehrere Aktuatoren 32a, 32b gehören. Beispielsweise können dies longitudinal arbeitende Piezoaktoren sein, die z.B. gegensinnig angesteuert werden, um der Düse 20 eine Mikroschwenkbewegung oder eine Querbewegung zu erteilen.

Eine weitere Ausführungsform eines Instruments 12 ergibt sich aus Figur 6. Diese Ausführungsform ist sowohl als offenchirurgisches Instrument 12 nach Figur 1 wie auch als laproskopisches Instrument nach Figur 4 anwendar. Der Träger 33 ist z.B. als flexibler Schlauch 37 ausgebildet und enthält den Kanal 15. Das distale Ende des Schlauchs 37 ist mit der Düse 20 verbunden. Dieser sind, wie Figur 7 zeigt, mehrere, z.B. vier, Aktuatoren 32a, 32b, 32c, 32d zugeordnet, die der Düse 20 eine quer zur Längsachse gerichtete Bewegung erteilen können. Die Aktuatoren 32a - 32d sind bspw. als Piezoelemente ausgebildet, wobei jeweils einander gegenüberliegenden Piezoelemente 32 und 32b bzw. 32c und 32d durch entsprechende Ansteuersignale Uₓ und U_{y} (oder U'ₓ und U'_{y}) betätigt werden. Durch Bereitstellung entsprechender in Frequenz, Kurvenform und Phase aufeinander abgestimmter Ansteuersignale Uₓ und U_{y} können nahezu beliebige Strahlbilder erhalten werden.

Figur 6 veranschaulicht die Ausbildung eines Fächerstrahls. Hierbei werden lediglich zwei einander gegenüber liegende Aktuatoren 32a, 32b so angesteuert, dass die Düse 20 quer zur Hauptaustrittsrichtung und Längsachse 28 schwingt. Die Querschwingung kann dabei eine relativ geringe Amplitude haben. Entscheidend ist die Schnelle der Schwingung (d.h. die (maximale) Geschwindigkeit der Düse in Querrichtung), die den quer zur Längsrichtung 28 gerichteten Impulsanteil bestimmt. Figur 6 veranschaulicht die Impulsanteile p_{L} für die Längsrichtung und p_{Q} für die Querrichtung. Wie ersichtlich, ist der längs gerichtete Impulsanteil p_{L} im Wesentlichen konstant. Der quer gerichtete Impulsanteil p_{Q} schwingt jedoch, d.h. er ist zeitabhängig. Auf diese Weise wird ein Fächerstrahl erzeugt, dessen Öffnungswinkel α durch die Größe und Frequenz der Amplitude des Ansteuersignals bestimmt werden kann.

Wird die Düse 20 z.B. durch Ansteuerung des anderen Aktuatorpaars 32c, 32d auf einem Kreis geführt, entsteht entsprechend ein Hohlkegelstrahl. Auch die Erzeugung eines Vollkegelstrahls ist möglich, wenn die in ihrer Schwingung 90° gegeneinander versetzten Ansteuersignal Uₓ und U_{y} z.B. nicht mit konstanter Amplitude, sondern mit einer Sägezahnmodulation bereitgestellt werden. Das Ergebnis zeigt Figur 9. Der quer gerichtete Impuls p_{Q} wird durch einen umlaufenden Vektor beschrieben, dessen Länge sich periodisch von Maximum auf Null reduziert.

Die Ansteuersignale Uₓ und U_{y} für das Aktuatorpaar 32a, 32b und/oder 32b, 32c können mittels der Bedienelemente 23 und/oder 24 verändert werden, um die Strahlform zu ändern. Z.B. zeigt Figur andere Kurvenformen U'ₓ und U'_{y} zur Erzeugung anderer Strahlformen. Die Kurvenformen U'ₓ und U'_{y} können sich von den Kurvenformen Uₓ und U_{y} in verschiedenen Parametern, wie Kurvenform, Amplitude, Frequenz u.a. unterscheiden. Solche Parameter können über die Bedienelemente 23 und/oder 24 abgerufen oder eingestellt werden.

Wie ersichtlich, sind durch verschiedene Signalformen verschiedene Grundcharakteristika einstellbar. Werden die Piezoaktoren, bspw. mit von der Sinusform abweichenden Kurvenformen (Rechteckschwingung, Sägezahnschwingung, Dreieckschwingung, Trapezschwingung oder dergleichen) beaufschlagt, können Fächerstrahlen, die randbetont arbeiten, Fächerstrahlen, die zentrumsbetont wirken, Hohlkegelstrahlen, die einen nicht kreisförmigen Querschnitt (z.B. Quadratquerschnitt mit eckenbetonter Strahlwirkung oder sonstige Eigenschaften) haben.

Erfindungsgemäß wird ein fluidchirurgisches Instrument bereitgestellt, das eine schwingungsbeaufschlagbare Düse 20 aufweist. Diese Grundkonzept kann dazu genutzt werden, während des Betriebs der Düse die Strahlrichtung zu ändern, um eine künstliche Wischbewegung zu erzeugen oder um der Düse lediglich Mikrobewegungen zu erteilen, die in den ausgestoßenen Fluidstrahl einen Querimpuls einleiten und so zu einer Veränderung des Strahlbilds führen. Es ist möglich, beide Effekte miteinander zu kombinieren. Auf diese Weise kann der Chirurg bspw. softwaretechnisch bereitgestellte verschiedene Strahlformen auf Befehl abrufen und mit ein und demselben Instrument erzeugen. Er kann somit verschiedene Wirkungen erzielen, ohne das Instrument und die Düse wechseln zu müssen.

### Bezugszeichenliste:

- 10: System
- 11: Organ
- 12: Instrument
- 13: Leitung
- 14: Gerät
- 15: Fluidkanal, Fluidleiter
- 16: Leitungen
- 17: Handgriff
- 18: Applikator
- 19: distales Ende
- 20: Düse
- 21: Strahl
- 22: Bedienelement
- 23, 24: Bedienelemente / Auswahleinrichtung
- 25: Einrichtung
- 26: proximales Ende der Leitung 13
- 27: Ansteuereinrichtung
- 28 - 30: strichpunktierte Linien - Längsrichtung
- 31: schwenkbare Verbindung
- 32: Aktuator 32a - 32d
- 33: Träger
- 34: Balg
- 35: Endoskop
- 36: Rohrschaft
- 37: Schlauch
- 38: Generator

## Patentansprüche

1. Fluidchirurgisches Instrument (12), insbesondere endoskopisches Instrument,
mit einem Fluidleiter (15), der sich durch einen Träger (33) erstreckt und an einem proximalen Ende (26) mit einem unter Druck stehenden Fluid beaufschlagbar ist,
mit einer Düse (20), die mit einem distalen Ende (19) des Fluidleiters (15) verbunden ist, um einen Strahl (21) des von dem Fluidleiter (15) der Düse (20) zugeführten Fluids in einer Austrittsrichtung (28) austreten zu lassen,
mit einem Aktuator (32), der mit der Düse (20) in Wirkverbindung steht, um diese in Bezug auf den Träger (33) oszillierend zu bewegen.

2. Instrument nach Anspruch 1, **dadurch gekennzeichnet, dass** die Düse (20) an dem distalen Ende (26) des Trägers (33) angeordnet ist.

3. Instrument nach Anspruch 2, **dadurch gekennzeichnet, dass** der Aktuator (32) zwischen der Düse (20) und dem Träger (33) angeordnet ist.

4. Instrument nach Anspruch 2, **dadurch gekennzeichnet, dass** die Düse (20) an dem Träger (33) elastisch gelagert ist.

5. Instrument nach einem der vorstehenden Ansprüche, **dadurch gekennzeichnet, dass** der Aktuator (32) dazu eingerichtet ist, die Düse (20) um mindestens eine Achse, die quer zu der Austrittsrichtung (28) orientiert ist, zu schwenken und/oder entlang einer Achse zu bewegen, die parallel zur Austrittsrichtung (28) angeordnet ist.

6. Instrument nach einem der vorstehenden Ansprüche, **dadurch gekennzeichnet, dass** der Aktuator (32) dazu eingerichtet ist, die Düse (20) um zwei Achsen, die quer zu der Austrittsrichtung (28) orientiert sind, zu schwenken und/oder die entlang einer Achse zu bewegen, die parallel zur Austrittsrichtung (28) angeordnet ist.

7. Instrument nach einem der vorstehenden Ansprüche, **dadurch gekennzeichnet, dass** der Aktuator (32) mindestens einen Piezoantrieb aufweist.

8. Instrument nach einem der vorstehenden Ansprüche, **dadurch gekennzeichnet, dass** der Aktuator (32) mindestens einen Vibramotor aufweist.

9. Instrument nach einem der vorstehenden Ansprüche, **dadurch gekennzeichnet, dass** der Aktuator (32) mit einer Ansteuereinrichtung (27) verbunden ist, um dem Aktuator (32) von der Ansteuereinrichtung (27) erzeugte Ansteuersignale (Uₓ, U_{y}) zuzuleiten.

10. Instrument nach Anspruch 9, **dadurch gekennzeichnet, dass** die Ansteuereinrichtung (27) zur Erzeugung eines periodischen Ansteuersignals (Uₓ, U_{y}) eingerichtet ist.

11. Instrument nach Anspruch 9 oder 10, **dadurch gekennzeichnet, dass** die Ansteuereinrichtung (27) zur Erzeugung wenigstens zweier verschiedener Ansteuersignale (Uₓ, U_{y}) eingerichtet und mit einer Auswahleinrichtung (23, 24) versehen ist, mittels derer die Ansteuereinrichtung (27) von der Erzeugung eines Ansteuersignals (Uₓ, U_{y}) auf die Erzeugung eines anderen Ansteuersignals (U'ₓ, U'_{y}) umschaltbar ist.

12. Instrument nach Anspruch 11, **dadurch gekennzeichnet, dass** ein erstes der Ansteuersignale (Uₓ, U_{y}) einem ersten Strahlbild und ein zweites der Ansteuersignale (U'ₓ, U'_{y}) einem zweiten Strahlbild zugeordnet ist.

13. Instrument nach einem der vorstehenden Ansprüche, **dadurch gekennzeichnet, dass** die Düse (20) mit einer elektrischen Leitung (16) verbunden ist, die mit einem HF-Generator (38) verbunden ist, um der Düse HF-Leistung zuzuführen.

14. Instrument nach einem der vorstehenden Ansprüche, **dadurch gekennzeichnet, dass** der Düse (20) eine Einrichtung (25) zur Erzeugung von Druckimpulsen vorgeschaltet ist.

15. Instrument nach einem der vorstehenden Ansprüche, **dadurch gekennzeichnet, dass** der Aktuator (32) eine vom Instrument (12) trennbare Einheit bildet.
